# EUROPÄISCHE PATENTANMELDUNG

(11) **EP 2 700 395 A2**
(43) Veröffentlichungstag der Anmeldung: **26.02.2014**
(21) Anmeldenummer: 13167851.8
(22) Anmeldetag: 15.05.2013
(51) Int. Cl.: A61K 8/34, A61K 8/67, A61Q 11/00, A61K 8/19

(54) **Lactoperoxidase-aktivierendes Mund- und Zahnpflege- und Reinigungsmittel**

(30) Priorität: 29.06.2012 DE 102012211347
(71) Anmelder: Henkel AG&Co. KGAA, 40589 Düsseldorf (DE)
(72) Erfinder: Giesen, Melanie, 47608 Geldern (DE); Miehlich, Kristin, 42119 Wuppertal (DE); Zelic, Daniela, 45239 Essen (DE); Heinen, Gudrun, 40215 Düsseldorf (DE)

(57) **Zusammenfassung**

Mund- und Zahnpflege- und -reinigungsmittel, die- jeweils bezogen auf ihr Gewicht - 0,001 bis 5 Gew.-% Niacinamid und/oder Nicotinsäure, 1 bis 70 Gew.-% Sorbit und/oder Glycerin und/oder 1,2-Propylenglycol und 0,01 bis 5 Gew.% Fluorverbindung(en) enthalten, führen den Nutzen von Lactoperoxidase herbei, ohne Enzyme im Mund- und Zahnpflege- und -reinigungsmittel einsetzen zu müssen.

## Beschreibung

Die Erfindung betrifft Zubereitungen zur Mund- und Zahnpflege- und -reinigung, die aufgrund von speziellen Inhaltsstoffen eine verbesserte antibakterielle durch Stimulierung der köpereigenen Abwehrsysteme aufweisen.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Da die herkömmliche Zahnreinigung mittels Bürste insbesondere in den Zahnzwischenräumen und am Ende der Zahnreihe an ihre Grenzen stößt, ist die Verbesserung der interndentalen und approximalen Reinigungsleistung ein großes Bedürfnis auf dem Gebiet der Mund- und Zahnpflege-und -reinigungsmittel.

Neben seiner Funktion zur Befeuchtung der Mundhöhle bildet der humane Speichel durch bestimmte Inhaltsstoffe wie z.B. Lysozym, Immunglobulin A, Laktoferrin und Histatinein ein antibakterielles Abwehrsystem. Eine der wichtigsten Komponenten dieses Abwehrsystem ist die Lactoperoxiase (LPO), die von den Speicheldrüsen (Glandula parotidea und Glandula submandibularis) als Inhaltsstoff des humanen Speichels sekretiert wird. Ihre antibakterielle Wirkung entfaltet die LPO im Speichel durch die Katalyse der Oxidation von Thiocyanat (SCN⁻), welches ebenfalls im Speichel vorkommt, durch Wasserstoffperoxid zu Hypothiocyanat (OSCN⁻).

H₂O₂+SCN⁻→OSCN⁻+H2O

Das milde Oxidationsmittel Hypothiocyanat weist signifikante antibakterielle Eigenschaften auf, beispielsweise gegen Streprococcus mutans. Die antibakterielle Wirkung beruht auf der Oxidation von SH-Gruppen bakterieneigener Enzyme wie der Hexogenase und Glycerinaldehyd-3-phophatdehydrogenase. Dadurch wird der Glucosestoffwechsel und damit das Wachstum der Bakterien gehemmt. Insbesondere bei geringen Konzentrationen von Peroxid sind die antibakteriellen Eigenschaften des Lactoperoxidasesystems deutlich höher als die von Wasserstoffperoxid allein.

Zur Stärkung dieses Abwehrsystems ist es von Vorteil, die Menge oder Aktivität der Lactoperoxidase im Mund zu erhöhen. Zu diesem Zweck wurde Lactoperoxidase bereits in Zahnpasten oder Mundwässern verarbeitet. Neben der geringen Wirksamkeit dieser Systeme ist der direkte Einsatz von Enzymen in Zahnpflegeprodukten auch aus toxikologischer Sicht nicht immer wünschenswert und z.T aufgrund gesetzlicher Bestimmungen nicht erlaubt.

Der vorliegenden Erfindung lag die Aufgabe zugrunde, Mund- und Zahnpflege- und - reinigungsmittel bereitzustellen, die eine verbesserte interndentale und approximale Reinigungsleistung aufweisen. Dabei sollte auf den Einsatz toxikologisch bedenklicher Inhaltsstoffe verzichtet und eine Applikation unter möglichst vielen gesetzlichen Regelungen, d.h. in möglichst vielen Ländern, ermöglicht werden.

Es wurde nun gefunden, dass bestimmte Verbindungen in einer Feuchthaltemittel- und Fluoridhaltigen Matrix geeignet sind, den Nutzen von Lactoperoxidase herbeizuführen, ohne Enzyme im Mund- und Zahnpflege- und -reinigungsmittel einsetzen zu müssen.

Gegenstand der vorliegenden Erfindung sind in einer ersten Ausführungsform Mund- und Zahnpflege- und -reinigungsmittel, enthaltend -jeweils bezogen auf ihr Gewicht -
- 0,001 bis 5 Gew.-% Niacinamid und/oder Nicotinsäure,
- 1 bis 70 Gew.-% Sorbit und/oder Glycerin und/oder 1,2-Propylenglycol,
- 0,01 bis 5 Gew.% Fluorverbindung(en).

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes sowie Mund- und Zahngele. Bevorzugt geeignet sind Zahnpasten und flüssige Zahn-reinigungsmittel. Hierzu können die Mund- und Zahnpflege- und reinigungsmittel z.B. in Form von Zahnpasten, flüssigen Zahncremes, Zahnpulvern, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden.

Die erfindungsgemäßen Zubereitungen enthalten als ersten wesentlichen Inhaltstoff 0,001 bis 5 Gew.-% Niacinamid und/oder Nicotinsäure. Niacin (Vitamin B3) kommt in Lebensmitteln und in Supplementen in zwei Formen vor: Nikotinsäure und Niacinamid. Beide können vom Körper in die metabolisch aktive Form Niacin verwandelt werden. Vitamin B3 ist am Kohlenhydrat-, Protein- und Fettstoffwechsel, an der Energiegewinnung und bei Entgiftungs- und antioxidativen Systemen des Körpers beteiligt. Niacin ist nötig für die Funktion von über 200 Enzymen im ganzen Körper. Es ist beteiligt an der Biosynthese von Verbindungen wie Fettsäuren und Steroiden. Es ist außerdem unerlässlich bei der Energieproduktion. Niacin spielt auch eine bedeutende Rolle bei der Aufrechterhaltung der Gesundheit von Haut, Muskelgewebe, Nerven- und Verdauungssystem. Erfindungsgemäß bevorzugte Mittel enthalten Nicotinsäure und/oder Niacinamid in engeren Mengenbereichen. Bevorzugte Mittel sind dadurch gekennzeichnet, dass sie 0,01 bis 2,5 Gew.-%, vorzugsweise 0,02 bis 2 Gew.-%, weiter bevorzugt 0,05 bis 1,5 Gew.-% und insbesondere 0,1 bis 1 Gew.-% Niacinamid und/oder Nicotinsäure enthalten.

Die genannten Mengen beziehen sich auf die Gesamtmenge an Niacinamid und Nicotinsäure in den erfindungsgemäßen Zubereitungen, die Formulierung "und/oder" bedeutet dabei, dass erfindungsgemäße Mittel entsprechende Mengen an Niacinamid oder an Nicotinsäure oder an einer Mischung von Niacinamid und Nicotinsäure enthalten können. Besonders bevorzugt ist erfindungsgemäß der Einsatz von Niacinamid, wobei besonders bevorzugte erfindungsgemäße Mund- und Zahnpflege- und -reinigungsmittel 0,01 bis 2,5 Gew.-%, vorzugsweise 0,02 bis 2 Gew.-%, weiter bevorzugt 0,05 bis 1,5 Gew.-% und insbesondere 0,1 bis 1 Gew.-% Niacinamid enthalten.

Als Träger für die Zahnpasten, der die Einstellung einer geeigneten Konsistenz für die Dosierung aus Tuben, Spendebehältern oder flexiblen Flaschen ermöglicht, eignet sich beispielsweise eine Kombination aus Feuchthaltemitteln, Bindemitteln und Wasser. Feuchthaltemittel sind beispielsweise das in den erfindungsgemäßen Zusammensetzungen enthaltene Sorbit bzw. Glycerin bzw. 1,2-Propylenglycol.

Die erfindungsgemäßen Zusammensetzungen enthalten 1 bis 70 Gew.-% Sorbit und/oder Glycerin und/oder 1,2-Propylenglycol. Auch hier beziehen sich die genannten Mengen auf die Gesamtmenge an Sorbit, Glycerin und 1,2-Propylenglycol in den erfindungsgemäßen Zubereitungen, die Formulierung "und/oder" bedeutet dabei, dass erfindungsgemäße Mittel entsprechende Mengen an Sorbit oder an Glycerin oder an 1,2-Propylenglycol oder an einer Mischung von Sorbit und Glycerin oder an einer Mischung von Sorbit und 1,2-Propylenglycol oder an einer Mischung von Glycerin und 1,2-Propylenglycol oder an einer Mischung von Glycerin und Sorbit und 1,2-Propylenglycol enthalten können. Besonders bevorzugt ist der Einsatz innerhalb engerer Mengenbereiche, wobei bevorzugte erfindungsgemäße Mund- und Zahnpflege-und -reinigungsmittel dadurch gekennzeichnet sind, dass sie 12 bis 70 Gew.-%, vorzugsweise 15 bis 60 Gew.-%, besonders bevorzugt 17 bis 55 Gew.-% und insbesondere 20 bis 50 Gew.-% Sorbit und/oder Glycerin und/oder 1,2-Propylenglycol enthalten.

Neben diesen Stoffen, von denen mindestens einer in einer Menge von mindestens 1 Gew.-% in den erfindungsgemäßen Mitteln enthalten ist, eignen sich als Feuchthaltemittel vorzugsweise Alkohole mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt. Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden.

Wichtige Vertreter von Polyhydroxyverbindungen mit 2 OH-Gruppen sind auch die Polyethylen-und Polypropylenglycole. Als bevorzugte weitere Feuchthaltemittel können z. B. Xylit, Propylenglycole, Polyethylenglycole, insbesondere solche mit mittleren Molekulargewichten von 200-800 eingesetzt werden. Das bzw. die zusätzlichen Feuchthaltemittel können in den erfindungsgemäßen Mitteln in variierenden Mengen eingesetzt werden. Während in Abhängigkeit von den übrigen optionalen Inhaltsstoffen Mengen von wenigen % (beispielsweise 1, 2, 3, 4, 5, 6, 7, 8, 9, 10 Gew.%, jeweils bezogen auf das gesamte Mittel) enthalten sein können, existieren auch Formulierungen, die deutlich höhere Feuchthaltemittel-Gehalte aufweisen. Der Gesamt-Feuchtmittel-Gehalt (einschließlich des in den erfindungsgemäßen Mitteln enthaltenen Sorbits, Glycerins und 1,2-Propylenglycols) beträgt beispielsweise 50, 55, 60, 65, 70, 75 Gew.%, jeweils bezogen auf das gesamte Mittel. Üblich und bevorzugt sind erfindungsgemäße Mittel, die (einschließlich des in den erfindungsgemäßen Mitteln enthaltenen Sorbits, Glycerins und 1,2-Propylenglycols) zwischen 10 und 75 Gew.% Feuchthaltemittel enthalten. Gehalte zwischen 15 und 70, vorzugsweise zwischen 20 und 60 Gew.%, jeweils bezogen auf das gesamte Mittel, können bevorzugt sein.

Die erfindungsgemäßen Zusammensetzungen enthalten als dritten wesentlichen Inhaltsstoff 0,01 bis 5 Gew.% Fluorverbindung(en). Diese Antikaries-Wirkstoffe, die die Wirkung des Niacinamids steigern können, können beispielsweise aus organischen oder anorganischen Fluoriden ausgewählt sein, z. B. aus Natriumfluorid, Kaliumfluorid, Natriummonofluorphosphat und Natriumfluorosilikat. Auch Zinkfluorid, Zinn-(II)-fluorid sind bevorzugt. Bevorzugt sollte eine Menge von 0,01 - 0,2 Gew.-% Fluor in Form der genannten Verbindungen enthalten sein.

Erfindungsgemäß bevorzugte Mund- und Zahnpflege- und -reinigungsmittel sind dadurch gekennzeichnet, dass sie 0,05 bis 4,5 Gew.%, vorzugsweise von 0,1 bis 2,5 Gew.% und insbesondere von 0,2 bis 1,1 Gew.% Fluorverbindung(en) aus der Gruppe Natriumfluorid, Kaliumfluorid, Natriummonofluorphosphat, Zinkfluorid, Zinnfluorid und Natriumfluorosilikat enthalten.

Die erfindungsgemäßen Mund- und Zahnpflege- und reinigungsmittel, insbesondere die Zahnpasten, können z. B. auch antimikrobielle Stoffe als Konservierungsmittel oder als Antiplaque-Wirkstoffe enthalten. Derartige Stoffe können z. B. ausgewählt sein aus p-Hydroxybenzoesäuremethyl-, -ethyl- oder propylester, Natriumsorbat, Natriumbenzoat, Bromchlorophen, Triclosan, Phenyl- Salicylsäureester, Biguanide z. B. Chlorhexidin, Thymol usw.. Erfindungsgemäß bevorzugte Mund- und Zahnpflege- und -reinigungsmittel sind dadurch gekennzeichnet, dass sie zusätzlich Antiplaque-Wirkstoffe, vorzugsweise p-Hydroxybenzoesäuremethyl-, -ethyl- oder propylester, Natriumsorbat, Natriumbenzoat, Bromchlorophen, Triclosan, Phenyl- Salicylsäureester, Biguanide z. B. Chlorhexidin, Thymol, vorzugsweise in Mengen von 0,1 bis 5 Gew.%, vorzugsweise von 0,25 bis 2,5 Gew.% und insbesondere von 0,5 bis 1,5 Gew.%, jeweils bezogen auf das gesamte Mittel, enthalten.

Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Die erfindungsgemäßen Mittel, insbesondere die Zahnpasten oder flüssigen Zahncremes enthalten vorzugsweise ein oder mehrere Poliermittel, üblicherweise in einer Menge von 5 bis 50 Gew.-%.

Als Poliermittel eignen sich prinzipiell alle für Zahnpasten bekannten Reibkörper, insbesondere solche, die keine Calciumionen enthalten. Bevorzugt geeignete Poliermittel-komponenten sind daher Kieselsäuren, Aluminiumhydroxid, Aluminiumoxid, Natrium-aluminiumsilikate, organische Polymere oder Gemische solcher Reibkörper.

Calciumhaltige Polierkomponenten wie z.B. Kreide, Calciumpyrophosphat, Dicalcium-phosphatdihydrat können aber in Mengen bis zu 5 Gew.-% enthalten sein.

Der Gesamtgehalt an Poliermitteln liegt vorzugsweise im Bereich von 5 - 50 Gew.-% des Zahnpflegemittels.

Besonders bevorzugt sind Zahnpasten und flüssige Zahnreinigungsmittel, die als Polier-mittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalt von 15 bis 35 Gew.-%, so werden die sogenannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m2/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident12 DS (DEGUSSA) erhältlich.

Andere Fällungskieselsäuren dieser Art sind Sident 8 (DEGUSSA) und Sorbosil AC 39 (Crosfield Chemicals). Diese Kieselsäuren zeichnen sich durch eine geringere Verdickungswirkung und eine etwas höhere mittlere Teilchengröße von 8 --14 µm bei einer spezifischen Oberfläche von 40 - 75 m2/g (nach BET) aus und eignen sich besonders gut für flüssige Zahncremes. Diese sollten eine Viskosität (25°C, Scherrate D = 10 s-1) von 10 - 100 Pa.s aufweisen.

Zahnpasten, die eine deutlich höhere Viskosität von mehr als 100 Pa.s (25° C, D = 10 s-1) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, sogenannte Verdickungs-kieselsäuren mit einer BET-Oberfläche von 150 --250 m2/g zu, z.B. die Handelsprodukte Sipernat 22 LS oder Sipernat 320 DS.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper,vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Die Mund- und Zahnpflege- und reinigungsmittel, insbesondere die Zahnpasten, können z.B. auch Substanzen enthalten, die gegen Zahnstein wirksam sind. Derartige Substanzen können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P₂O₇, Na₂K₂P₂O₇, Na₂H₂P₂O₇ und K₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.

Erfindungsgemäß bevorzugte Mund- und Zahnpflege- und -reinigungsmittel sind dadurch gekennzeichnet, dass sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Auch oberflächenaktive Substanzen sind in den Zahnpasten zur Unterstützung der Reinigungswirkung und gewünschtenfalls auch zur Entwicklung von Schaum beim Zähnebürsten sowie zur Stabilisierung der Polierkörperdispersion im Träger in einer Menge von 0,1-5 Gew.-% enthalten.

Geeignete Tenside sind z. B. lineare Natriumalkylsulfate mit 12-18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12-16 C-Atomen in der linearen Alkylgruppe und 2-6 Glycolethergruppen im Molekül, von linearem Alkan(C₁₂- C₁₈)-sulfonat, von Sulfobernsteinsäuremonoalkyl(C₁₂-C₁₈)-estern, von sulfatisierten Fettsäuremonoglyceriden, sulfatisierten Fettsäurealkanolamiden, Sulfoessigsäurealkyl(C₁₂-C₁₆ )-estern, Acylsarcosinen, Acyltauriden und Acylisothionaten mit jeweils 8-18 C-Atomen in der Acylgruppe. Auch zwitterionische, ampholytische und nichtionische Tenside sind geeignet, z. B. Oxethylate von Fettsäuremono- und -diglyceriden, von Fettsäure-Sorbitanestern und Alkyl(oligo)-Glucoside.

Die Mund- und Zahnpflegemittel, insbesondere die Zahnpasten, können auch die Unempfindlichkeit der Zähne steigernde Substanzen enthalten, beispielsweise Kaliumsalze wie z. B. Kaliumnitrat, Kaliumcitrat, Kaliumchlorid, Kaliumbicarbonat und Kaliumoxalat. Erfindungsgemäß bevorzugte Mund- und Zahnpflege- und -reinigungsmittel sind dadurch gekennzeichnet, dass sie die Unempfindlichkeit der Zähne steigernde Substanzen, vorzugsweise Kaliumsalze, besonders bevorzugt Kaliumnitrat und/oder Kaliumcitrat und/oder Kaliumchlorid und/oder Kaliumbicarbonat und/oder Kaliumoxalat, vorzugsweise in Mengen von 0,5 bis 20 Gew.%, besonders bevorzugt von 1,0 bis 15 Gew.%, weiter bevorzugt von 2,5 bis 10 Gew.-% und insbesondere von 4,0 bis 8,0 Gew.%, jeweils bezogen auf das gesamte Mittel, enthalten.

Die erfindungsgemäßen Mittel, insbesondere die Zahnpasten, können auch zusätzlich weitere wundheilende und entzündungshemmende Stoffe, z. B. Wirkstoffe gegen Zahnfleischentzündungen, enthalten. Derartige Stoffe können z. B. ausgewählt sein aus Allantoin, Azulen, Kamillenextrakten, Tocopherol, Panthenol, Bisabolol, Salbeiextrakten.

Als nicht-kationische, bakterizide Komponente eignen sich z.B. Phenole, Resorcine, Bisphenole, Salicylanilide und deren halogenierte Derivate, halogenierte Carbanilide und p-Hydroxybenzoesäureester. Besonders bevorzugte antimikrobielle Komponenten sind halogenierte Diphenylether, z.B. 2,4-Dichlor-2'-hydroxydiphenylether, 4,4'-Dichlor-2'-hydroxydiphenylether, 2,4,4'-Tribrom-2'-hydroxydiphenylether und 2,4,4'-Trichlor-2'-hydroxydiphenylether (Triclosan). Sie werden bevorzugt in Mengen von 0,01 - 1 Gew.-% in die erfindungsgemäßen Zahnpflegemittel eingesetzt. Besonders bevorzugt wird Triclosan in einer Menge von 0,01 - 0,3 Gew.-% eingesetzt.

D-Panthenol D - (+) - 2,4-Dihydroxy-N-(3-hydroxypropyl)-3,3-dimethyl-butyramid zeigt eine der Pantothensäure entsprechende biologische Aktivität. Die Pantothensäure (R - (+) - N - (2,4-Dihydroxy-3,3-dimethylbutyryl-ß-alanin) ist eine Vorstufe in der Biosynthese des Coenzyms A und wird zum Vitamin-B-Komplex (B3) gezählt. Diese Stoffe sind dafür bekannt, dass sie die Wundheilung fördern und eine günstige Wirkung auf die Haut haben. Sie sind daher auch gelegentlich in Zahnpasten beschrieben worden. Die erfindungsgemäßen Zahnpflegemittel enthalten bevorzugt 0,05 - 5 Gew.-% Panthenol oder ein Salz der Pantothensäure.

Retinol (3,7-Dimethyl-9-(2,6,6-trimethyl-1-cyclohexenyl)-2,4,6,8-nonatetraen-1-ol ist der internationale Freiname für Vitamin A1. Anstelle des Retinols kann auch eines seiner Derivate mit ähnlicher biologischer Wirkung, z.B. ein Ester oder die Retinoesäure (Tretinoin), eines ihrer Salze oder ihre Ester verwendet werden. Bevorzugt wird ein Retinol-Ester, insbesondere ein Fettsäureester einer Fettsäure mit 12 - 22 C-Atomen verwendet. Besonders bevorzugt ist Retinol-Palmitat geeignet. Bei Verwendung eines Retinol-Esters, z.B. Retinol-Palmitat mit einer Aktivität von 1,7 ˙ 10⁶ I.E. pro g ist eine Menge von 0,001 bis 0,1 Gew.-% bevorzugt. Bei Verwendung anderer Retinol-Derivate empfiehlt sich eine Einsatzmenge, die einer Konzentration von 10³ bis 10⁶ I.E. (Internationale Einheiten) pro 100 g entspricht.

Bevorzugte Zahnpflegemittel gemäß der vorliegenden Erfindung enthalten neben Poliermitteln, Fluorverbindungen, Feuchthaltemitteln und Bindemitteln bevorzugt
0,01 - 1 Gew.-% eines halogenierten Diphenylethers
0,05 - 5 Gew.-% Panthenol oder ein Salz der Pantothensäure und
0,01 - 0,1 Gew.-% eines Retinol-Esters, bevorzugt Retinol-Palmitat.

Die erfindungsgemäßen Mittel, insbesondere die Zahnpasten, können auch Substanzen zur Erhöhung des mineralisierenden Potentials enthalten, beispielsweise calciumhaltige Substanzen wie z. B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten.

Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose.

Weitere übliche Hilfs- und Zusatzstoffe für Zahnpasten sind
- Oberflächenaktive Stoffe, bevorzugt anionische, zwitterionische, amphotere, nichtionische Tenside oder eine Kombination mehrerer verschiedener Tenside
- Lösungsmittel und Lösungsvermittler, z.B. niedere einwertige oder mehrwertige Alkohole oder Ether, z.B. Ethanol, 1,2-Propylenglycol, Diethylenglycol oder Butyldi- glycol
- Pigmente, wie z.B. Titandioxid
- Farbstoffe
- Puffersubstanzen, z.B. primäre, sekundäre oder tertiäre Alkaliphosphate oder Citronensäure-/Na-Citrat
- weitere wundheilende oder entzündungshemmende Stoffe, z.B. Allantoin, Harnstoff, Azulen, Kamillewirkstoffe, Acetylsalicylsäurederivate oder Rhodanid
- weitere Vitamine wie z.B. Ascorbinsäure, Biotin, Tocopherol oder Rutin
- Mineralsalze wie z.B. Mangan-, Zink- oder Magnesiumsalze.

Eine weitere wichtige Gruppe von Inhaltstoffen, die in den erfindungsgemäßen Mitteln enthalten sein kann, sind die sogenannten bioaktiven Gläser.

Der Begriff "bioaktive Gläser" umfaßt im Rahmen der vorliegenden Anmeldung Gläser, welche biologisch wirksam und/oder biologisch aktiv sind. Die biologische Wirksamkeit eines Glases kann sich beispielsweise in dessen antimikrobiellen Eigenschaften zeigen, biologisch aktives Glas unterscheidet sich von herkömmlichen Kalk-Natrium-Silicat-Gläsern dadurch, dass es lebendes Gewebe bindet. Biologisch aktives Glas bezeichnet dabei beispielsweise ein Glas, das eine feste Bindung mit Körpergewebe eingeht, wobei eine Hydroxyl-Apatitschicht ausgebildet wird. Unter bioaktivem Glas wird auch ein Glas verstanden, das antimikrobielle und/oder entzündungshemmende Wirkung zeigt. Die Glaspulver zeigen gegenüber Bakterien, Pilzen sowie Viren eine biozide bzw. eine biostatische Wirkung; sind im Kontakt mit dem Menschen hautverträglich, toxikologisch unbedenklich und insbesondere auch zum Verzehr geeignet. Erfindungsgemäß besonders bevorzugte Mund- und Zahnpflege- und -reinigungsmittel sind dadurch gekennzeichnet, dass sie 0,2 bis 20 Gew.-%, vorzugsweise 0,4 bis 14 Gew.-%, besonders bevorzugt 0,5 bis 3 Gew.-% und insbesondere 0,6 bis 2 Gew.-% mindestens eines bioaktiven Glases enthalten.

Die erfindungsgemäßen Mund- und Zahnpflege- und -reinigungsmittel dieser Ausführungsform enthalten bioaktives Glas oder Glaspulver oder Glaskeramikpulver oder Kompositmaterialien, welche ein solches bioaktives Glas umfassen. Unter Glaspulvern werden im Rahmen der vorliegenden Anmeldung auch Granulate und Glaskügelchen verstanden.

Aufgrund der Anforderungen an die toxikologische Unbedenklichkeit des Glases sowie deren Eignung zum Verzehr soll das Glaspulver besonders rein sein. Die Belastung durch Schwermetalle ist vorzugsweise gering. So beträgt die Maximalkonzentration im Bereich der kosmetischen Formulierungen vorzugsweise für Pb < 20 ppm, Cd < 5 ppm, As < 5 ppm, Sb < 10 ppm, Hg < 1 ppm, Ni < 10 ppm.

Das unkeramisierte Ausgangsglas, das direkt in den bevorzugten erfindungsgemäßen Zusammensetzungen enthalten oder gegebenenfalls für die Herstellung einer erfindungsgemäß einsetzbaren Glaskeramik verwandt wird, enthält SiO₂ als Netzwerkbildner, vorzugsweise zwischen 35-80 Gew.-%. Bei niedrigeren Konzentrationen nimmt die spontane Kristallisationsneigung stark zu und die chemische Beständigkeit stark ab. Bei höheren SiO₂-Werten kann die Kristallisationsstabilität abnehmen, und die Verarbeitungstemperatur wird deutlich erhöht, so dass sich die Heißformgebungseigenschaften verschlechtern. Na₂O wird als Flußmittel beim Schmelzen des Glases eingesetzt. Bei Konzentrationen kleiner 5% wird das Schmelzverhalten negativ beeinflußt. Natrium ist Bestandteil der sich bei der Keramisierung bildenden Phasen und muß, sofern hohe kristalline Phasenanteile durch die Keramisierung eingestellt werden sollen, in entsprechend hohen Konzentrationen im Glas enthalten sein. K₂O wirkt als Flußmittel beim Schmelzen des Glases. Außerdem wird Kalium in wässrigen Systemen abgegeben. Liegen hohe Kaliumkonzentrationen im Glas vor, werden kaliumhaltige Phasen wie Kalzium-Silicaten ebenfalls ausgeschieden. Über den P₂O₅-Gehalt kann bei silikatischen Gläsern, Glaskeramiken oder Kompositen die chemische Beständigkeit des Glases und damit die Ionenabgabe in wässrigen Medien eingestellt werden. Bei Phospahtgläsern ist P₂O₅ Netzwerkbilder. Der P₂O₅-Gehalt liegt vorzugsweise zwischen 0 und 80 Gew.-%. Um die Schmelzbarkeit zu verbessern, kann das Glas bis zu 25 Gew.- % B₂O₃ enthalten. Al₂O₃ wird genutzt, um die chemische Beständigkeit des Glases einzustellen.

Zur Verstärkung der antimikrobiellen, insbesondere der antibakteriellen Eigenschaften der Glaskeramik können antimikrobiell wirkende Ionen wie z. B. Ag, Au, I, Ce, Cu, Zn in Konzentrationen kleiner 5 Gew.-% enthalten sein.

Farbgebende Ionen wie z. B. Mn, Cu, Fe, Cr, Co, V, können einzeln oder kombiniert, vorzugsweise in einer Gesamtkonzentration kleiner 1 Gew.-%, enthalten sein.

Üblicherweise wird das Glas bzw. die Glaskeramik in Pulverform eingesetzt. Die Keramisierung kann entweder mit einem Glasblock bzw. Glasribbons erfolgen oder aber mit Glaspulver. Nach der Keramisierung müssen die Glaskeramikblöcke oder Ribbons zu Pulver gemahlen werden. Wurde das Pulver keramisiert, muß gegebenenfalls auch erneut gemahlen werden, um Agglomerate, die während des Keramisierungsschrittes entständen sind, zu entfernen. Die Mahlungen können sowohl trocken als auch in wässrigen oder nicht wässrigen Mahlmedien durchgeführt werden. Üblicherweise liegen die Partikelgrößen kleiner 500 µm. Als zweckmäßig haben sich Partikelgrößen < 100 µm bzw. < 20 µm erwiesen. Besonders geeignet sind Partikelgrößen < 10 µm sowie kleiner 5 µm sowie kleiner 2 µm, siehe weiter unten.

Die in den bevorzugten erfindungsgemäßen Zusammensetzungen enthaltenen bioaktiven Gläser bzw. Glaspulver oder Glaskeramikpulver oder Komposit-Zusammensetzungen umfassen Gläser, die bevorzugt nachfolgende Komponenten umfassen: SiO₂: 35-80 Gew.-%, Na₂O: 0-35 Gew.- %, P₂O₅: 0-80 Gew.-%, MgO: 0-5 Gew.-%, Ag₂O: 0-0,5 Gew.-%, AgJ: 0-0,5 Gew.- %, NaJ: 0-5 Gew.-%, TiO₂: 0-5 Gew.-%, K₂O: 0-35 Gew.-%, ZnO: 0-10 Gew.-%, Al ₂O₃: 0-25 Gew.-% und B₂O₃: 0-25 Gew.-%.

Weiterhin können dem Grundglas gemäß obiger Zusammensetzung zur Erzielung weiterer Effekte wie beispielsweise Farbigkeit oder UV-Filterung Ionen wie Fe, Co, Cr, V, Ce, Cu, Mn, Ni, Bi, Sn, Ag, Au, J einzeln oder in Summe bis zu 10 Gew.-% zugegeben werden. Eine weiter Glaszusammensetzung kann wie folgt sein: SiO₂: 35-80 Gew.-%, Na₂O: 0-35 Gew.-%, P₂O₅: 0-80 Gew.-%, MgO: 0-5 Gew.- %, Ag₂O: 0-0,5 Gew.-%, AgJ: 0-0,5 Gew.-%, NaJ: 0-5 Gew.-%, TiO₂: 0-5 Gew.-%, K₂O: 0-35 Gew.-%, ZnO: 0-10 Gew.-%, Al₂ O₃: 0-25 Gew.-%, B₂O₃: 0- 25 Gew.-%, SnO: 0-5 Gew.-%, CeO₂: 0-3 Gew.- % und Au: 0,001-0,1 Gew.-%.

Besonders bevorzugte erfindungsgemäße Mund- und Zahnpflege- und -reinigungsmittel sind dadurch gekennzeichnet, dass das bioaktive Glas - bezogen auf sein Gewicht - folgende Zusammensetzung aufweist:

| | | | |
|---|---|---|---|
| SiO₂ | 35 bis 60 Gew.-%, | vorzugsweise | 40 bis 60 Gew.-%, |
| Na₂O | 0 bis 35 Gew.-%, | vorzugsweise | 5 bis 30 Gew.-%, |
| K₂O | 0 bis 35 Gew.-%, | vorzugsweise | 0 bis 20 Gew.-%, |
| P₂O₅ | 0 bis 10 Gew.-%, | vorzugsweise | 2 bis 10 Gew.-%, |
| MgO | 0 bis 10 Gew.-%, | vorzugsweise | 0 bis 5 Gew.-%, |
| CaO | 0 bis 35 Gew.-%, | vorzugsweise | 5 bis 30 Gew.-%, |
| Al₂O₃ | 0 bis 25 Gew.-%, | vorzugsweise | 0 bis 5 Gew.-%, |
| B₂O₃ | 0 bis 25 Gew.-%, | vorzugsweise | 0 bis 5 Gew.-%, |
| TiO₂ | 0 bis 10 Gew.-%, | vorzugsweise | 0,1 bis 5 Gew.-%. |

Wie bereits weiter oben erwähnt, wird das bioaktive Glas vorzugsweise in partikulärer Form eingesetzt. Hier sind besonders bevorzugte erfindungsgemäße Mund- und Zahnpflege- und - reinigungsmittel dadurch gekennzeichnet, dass das antimikrobielle Glas Teilchengrößen < 10 µm, vorzugsweise von 0,5 bis 4 µm, besonders bevorzugt von 1 bis 2 µm, aufweist.

Die erfindungsgemäßen Zusammensetzungen dieser Ausführungsform eigen sich hervorragend zur Zahnreinigung und -pflege. Sie reinigen die Zähne schonend und haben dabei den Zusatznutzen, dass sie die Zahnoberfläche glätten.

Ein weiterer Gegenstand der vorliegenden Erfindung ist die Verwendung von Niacinamid und/oder Nicotinsäure zur Steigerung der Lactoperoxidaseaktivität im Mundraum.

Ein weiterer Gegenstand der vorliegenden Erfindung ist die Verwendung von Niacinamid und/oder Nicotinsäure in Mund- und Zahnpflege- und -reinigungsmitteln zur Steigerung der Lactoperoxidaseaktivität.

Besonders bevorzugte Verwendungen sind dadurch gekennzeichnet, dass die Verwendung mittels eines erfindungsgemäßen Mund- und Zahnpflege- und -reinigungsmittels erfolgt.

Bezüglich weiterer bevorzugter Verwendungen gilt mutatis mutandis das zu den erfindungsgemäßen Mitteln Gesagte.

### Beispiel 1: Bestimmung des Einflusses von Niacinamid auf die Lactoperoxidaseaktivität im humanen Speichel

Fur Bestimmung der Lactoperoxidase im Speichel wurde mit Hilfe einer Salivette von 5 Probanden Speichel gewonnen, dieser abzentifugiert, ein Aliquot des Überstandes mit Niacinamid versetzt und 1h-24h bei 37°C inkubiert. Zur Messung werden die Proben mit TMB (3,3',5,5'-Tetramethylbenzidin) versetzt, das als Substrat des Enzyms Lactoperoxidase dient und von dieser zu einem blauen Farbstoff umgesetzt. Dieser kann spektroskopisch quantifiziert werden.

Die Tabelle zeigt die Aktivität der Lactoperoxidase nach Inkubation mit dem Rohstoff im Vergleich zum unbehandelten Speichel. Die Werte der 5 Probanden wurden gemittelt und der *standard error of the mean ermittelt.* Die Signifikanz wurde mit Hilfe des *student t-test* festgestellt.

Der unbehandelte Speichel zum jeweiligen Inkubationszeitpunkt wurde als 100% gesetzt.

| Konz | 1h Kontrolle | | 1h + Niacinamid | | 24h Kontrolle | | 24h + Niacinamid | |
|---|---|---|---|---|---|---|---|---|
| | MW [%] | SEM [%] | MW [%] | SEM [%] | MW [%] | SEM [%] | MW [%] | SEM [%] |
| 1% | 100 | 19,4 | 108,8 | 8,3 | 100 | 18,6 | 158,5 | 32,3 |
| 0,5% | 100 | 11,7 | 95,0 | 2,0 | 100 | 9,1 | 141,4 | 7,4 |
| | | | | | p-value = 0,046 | | | |

ie Ergebnisse zeigen, dass durch die Zugabe von 1% Niacinamid im Vergleich zur unbehandelten Kontrolle die Aktivität der Lactoperoxidase deutlich gesteigert werden kann. Dies gilt insbesondere für die Inkubationszeit von 24h. Bei einer Einsatzkonzentration von 0,5% Niacinamid ist die Steigerung der Lactoperoxidaseaktivität bei einer Inkubationszeit von 24h signifikant.

### Beispiel 2 Nachweis des kompensierenden Effektes von Niacinamid auf die Inhibition der Lactoperoxidaseaktivität im humanen Speichel durch NaF.

Um zu prüfen, ob Niacinamid auch einen schützenden Einfluss auf die Lactoperoxidaseaktivität hat, die durch bestimmte Substanzen inhibiert werden kann, wurde nach der Inkubation des Speichels mit Niacinamid noch NaF zur Probe gegeben und anschließend die Restaktivität der Lactoperoxidase bestimmt.

Die Tabelle zeigt die Aktivität der Lactoperoxidase nach Inkubation mit NaF und dem Rohstoff im Vergleich zum mit NaF behandelten Speichel. Die Werte der 5 Probanden wurden gemittelt und der *standard error of the mean* ermittelt. Die Signifikanz wurde mit Hilfe des *student t-test* festgestellt.

Der nur mit NaF behandelte Speichel diente als Kontrolle und wurde zum jeweiligen Inkubationszeitpunkt als 100% gesetzt.

| Konz | 1h Kontrolle NaF | | 1 h + NaF + Niacinamid | | 24h Kontrolle NaF | | 24h + Niacinamid + NaF | |
|---|---|---|---|---|---|---|---|---|
| | MW [%] | SEM [%] | MW [%] | SEM [%] | MW [%] | SEM [%] | MW [%] | SEM [%] |
| 1% | 100 | 9,1 | 117,5 | 4,3 | 100 | 19,6 | 143,3 | 8,3 |
| | p-value = 0,026 | | | | p-value = 0,014 | | | |
| 0,5% | 100 | 12,3 | 108,6 | 3,1 | 100 | 8,3 | 116 | 4,4 |
| | p-value = 0,048 | | | | p-value = 0,039 | | | |

Die Ergebnisse zeigen, dass durch die Zugabe von 0,5% und 1% Niacinamid im Vergleich zur mit NaF behandelten Kontrolle die Aktivität der Lactoperoxidase signifikant gesteigert werden kann.

| | |
|---|---|
| Beispiel 3 Zahnpastaformulierung | |
| Sorbitol | 45 - 70% |
| Hydrated silica | 5 - 20 % |
| Natriumflourid | 0,1 -0,32 % |
| Natrium Saccharin | 0,1 - 0,5 % |
| Norspermidin | 0,03%-0,1% |
| Aroma | 0,1 - 1,5 % |
| Ethanol | 0 - 5 % |
| Xanthan gum | 0,2 - 1 % |
| Anionic surfactant | 1 - 5 % |
| Cocamidopropyl Betaine | 0,5 - 1 % |
| Co Polymer (e.g. Gantrez) | 0,5 - 2 % |
| Water | ad 100% |
| | |
| Beispiel 4 Mundwasserformulierung | |
| Sorbitol | 1-5 % |
| PEG-60 Hydrogenated Castor Oil | 0,1 - 1 % |
| Natriumfluorid | 0,11 % |
| Trinatriumcitrat Dihydrat | 0,1- 0,6% |
| Citronensäure | 0,001- 0,01 % |
| Natrium Saccharin | 0,03 - 0,3% |
| Cetylpyridinium Chloride | 0,01 - 0,1 % |
| Aroma | 0,1 - 1% |
| Ethanaol | 0 - 30 % |
| Norspermidin | 0,03%-0,1% |
| Co Polymer (e.g. Gantrez) | 0,1 - 0,5 % |
| Water | ad 100% |

## Patentansprüche

1. Mund- und Zahnpflege- und -reinigungsmittel, enthaltend -jeweils bezogen auf sein Gewicht -
- 0,001 bis 5 Gew.-% Niacinamid und/oder Nicotinsäure,
- 1 bis 70 Gew.-% Sorbit und/oder Glycerin und/oder 1,2-Propylenglycol,
- 0,01 bis 5 Gew.% Fluorverbindung(en).

2. Mund- und Zahnpflege- und -reinigungsmittel gemäß Anspruch 1, **dadurch gekennzeichnet, dass** es 0,01 bis 2,5 Gew.-%, vorzugsweise 0,02 bis 2 Gew.-%, weiter bevorzugt 0,05 bis 1,5 Gew.-% und insbesondere 0,1 bis 1 Gew.-% Niacinamid enthält.

3. Mund- und Zahnpflege- und -reinigungsmittel nach einem der Ansprüche 1 oder 2, **dadurch gekennzeichnet, dass** es 12 bis 70 Gew.-%, vorzugsweise 15 bis 60 Gew.-%, besonders bevorzugt 17 bis 55 Gew.-% und insbesondere 20 bis 50 Gew.-% Sorbit und/oder Glycerin und/oder 1,2-Propylenglycol enthält.

4. Mund- und Zahnpflege- und -reinigungsmittel nach einem der Ansprüche 1 bis 3, **dadurch gekennzeichnet, dass** es 0,05 bis 4,5 Gew.%, vorzugsweise von 0,1 bis 2,5 Gew.% und insbesondere von 0,2 bis 1,1 Gew.% Fluorverbindung(en) aus der Gruppe Natriumfluorid, Kaliumfluorid, Natriummonofluorphosphat, Zinkfluorid, Zinnfluorid und Natriumfluorosilikat enthält.

5. Mund- und Zahnpflege- und -reinigungsmittel nach einem der Ansprüche 1 bis 4, **dadurch gekennzeichnet, dass** es zusätzlich Antiplaque-Wirkstoffe, vorzugsweise p-Hydroxybenzoesäuremethyl-, -ethyl- oder propylester, Natriumsorbat, Natriumbenzoat, Bromchlorophen, Triclosan, Phenyl- Salicylsäureester, Biguanide z. B. Chlorhexidin, Thymol, vorzugsweise in Mengen von 0,1 bis 5 Gew.%, vorzugsweise von 0,25 bis 2,5 Gew.% und insbesondere von 0,5 bis 1,5 Gew.%, jeweils bezogen auf das gesamte Mittel, enthält.

6. Mund- und Zahnpflege- und -reinigungs- oder Mund- und Zahnpflege-und -reinigungshilfsmittel nach einem der Ansprüche 1 bis 4, **dadurch gekennzeichnet, dass** es zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper,vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthält.

7. Mund- und Zahnpflege- und -reinigungsmittel nach einem der Ansprüche 1 bis 6, **dadurch gekennzeichnet, dass** es die Unempfindlichkeit der Zähne steigernde Substanzen, vorzugsweise Kaliumsalze, besonders bevorzugt Kaliumnitrat und/oder Kaliumcitrat und/oder Kaliumchlorid und/oder Kaliumbicarbonat und/oder Kaliumoxalat, vorzugsweise in Mengen von 0,5 bis 20 Gew.%, besonders bevorzugt von 1,0 bis 15 Gew.%, weiter bevorzugt von 2,5 bis 10 Gew.-% und insbesondere von 4,0 bis 8,0 Gew.%, jeweils bezogen auf das gesamte Mittel, enthält.

8. Verwendung von Niacinamid und/oder Nicotinsäure zur Steigerung der Lactoperoxidaseaktivität im Mundraum.

9. Verwendung von Niacinamid und/oder Nicotinsäure in Mund- und Zahnpflege-und -reinigungsmitteln zur Steigerung der Lactoperoxidaseaktivität.

10. Verwendung nach einem der Ansprüche 8 oder 9, **dadurch gekennzeichnet, dass** die Verwendung mitels eines Mund- und Zahnpflege- und -reinigungsmittels nach einem der Ansprüche 1 bis 7 erfolgt.
